# Europäisches Patentamt
# European Patent Office
# Office européen des brevets

(19)

(11) Veröffentlichungsnummer: **0 176 856**
**B1**

(12) **EUROPÄISCHE PATENTSCHRIFT**

(45) Veröffentlichungstag der Patentschrift: **23.01.91**

(51) Int. Cl.⁵: **C 07 D 217/20, C 07 B 57/00**

(21) Anmeldenummer: **85111715.0**

(22) Anmeldetag: **17.09.85**

(54) Verfahren zur Herstellung optisch aktiver Amine.

(30) Priorität: **03.10.84 DE 3436179**

(43) Veröffentlichungstag der Anmeldung:
**09.04.86 Patentblatt 86/15**

(45) Bekanntmachung des Hinweises auf die
Patenterteilung:
**23.01.91 Patentblatt 91/04**

(84) Benannte Vertragsstaaten:
**CH DE GB IT LI**

(56) Entgegenhaltungen:
**DE-A-2 003 486**

**CHEMICAL REVIEWS, Band 80, Nr. 3, Juni 1980,
Seiten 215-230, American Chemical Society; A.
COLLET et al.: "Optical resolution by direct
crystallization of enantiomer mixtures"**

(73) Patentinhaber: **MERCK PATENT GESELLSCHAFT
MIT BESCHRÄNKTER HAFTUNG**
**Frankfurter Strasse 250 Postfach 4119**
**D-6100 Darmstadt (DE)**

(72) Erfinder: **Wintermeyer, Willi, Dr.**
**Gartenstrasse 4**
**D-6104 Seeheim-Jugenheim (DE)**
Erfinder: **Reiff, Fritz, Dr.**
**Im Rassdorf 6**
**D-6104 Seeheim 1 (DE)**
Erfinder: **Müller, Hans-Rudolf, Dr.**
**Am Beckenwäldli 18**
**CH-8207 Schaffhausen (CH)**
Erfinder: **Conti, Josef**
**Winkelriedstrasse 22**
**CH-8200 Schaffhausen (CH)**

Courier Press, Leamington Spa, England.

**Beschreibung**

Die Erfindung betrifft ein Verfahren zur Herstellung von optisch aktivem 1-(4-Methoxybenzyl)-1,2,3,4,5,6,7,8-octahydroisochinolin.

Optisch aktive Enantiomere von Methoxybenzyloctahydroisochinolin sind wertvolle Zwischenprodukte für die Herstellung pharmakologisch wirksamer Verbindungen wie optisch aktiven 3-Methoxy-17-methylmorphinanen, die als Antitussivum und Analgetikum Verwendung finden.

Eine Enantiomerentrennung in einem früheren Stadium der Morphinansynthese und gegebenenfalls die Rückführung des unerwünschten Isomeren ist für die Wirtschaftlichkeit des Herstellungsverfahrens von großer Bedeutung.

Es sind bereits verschiedene Verfahren zur optischen Spaltung von (R,S)-1-(4-Methoxybenzyl)-octahydroisochinolin bekannt. Brossi und Schnider, Helv. Chim. Acta *39* (1965) 1376—86, beschreiben die Trennung durch fraktionierte Kristallisation der diastereomeren D-(-)-Mandelsäuresalze. Aus DBP 2003486 ist die Trennung in die Antipoden mit Hilfe optisch aktiver (-)-Di-O-isopropyliden-2-keto-L-gulonsaüre bekannt.

Diese Verfahren besitzen den Nachteil, daß zu ihrer Durch-führung ein teures optisch aktives Spaltungsreagenz benötigt wird, welches nur in einer enantiomeren Form zu Verfügung steht und sich nach erfolgter optischer Spaltung nur unvollständig wieder regenerieren läßt.

Es besteht somit ein Bedürfnis nach einem Spaltungsverfahren, welches ohne Verwendung eines optisch aktiven Hilfsreagenz die einfache und vollständige Gewinnung beider Enantiomeren erlaubt.

Diese Aufgabe wird gelöst durch das erfindungsgemäße Verfahren zur Herstellung von optisch aktivem 1-(4-Methoxybenzyl)-octahydroisochinolin durch Spontankristallisation der entsprechenden Acetate.

Gegenstand der Erfindung ist daher ein Verfahren zur Herstellung von optisch aktivem 1-(4-Methoxybenzyl)-1,2,3,4,5,6,7,8-octahydroisochinolin, dadurch gekennzeichnet, daß man die übersättigte Lösung von racemischem 1-(4-Methoxybenzyl)-octahydroisiochinoliniumacetat mit einer enantiomeren Form von 1-(4-Methoxybenzyl)-octahydroisochinoliniumacetat impft und das der Konfiguration der Impfkristalle entsprechende optisch aktive Acetat kristallisiert.

Ein weiterer Gegenstand der Erfindung ist die kontinuierliche Augestaltung des erfindungsgemäßen Verfahrens zum Zwecke der Gewinnung beider Enantiomeren nebeneinander.

Gegenstand der Erfindung sind auch (R,S)-1-(4-Methoxybenzyl)-1,2,3,4,5,6,7,8-octahydroisochinoliniumacetat, (R)-1-(4-Methoxybenzyl)-1,2,3,4,5,6,7,8-octahydroisochinoliniumacetat und (S)-1-(4-Methoxybenzyl)1,2,3,4,5,6,7,8-octahydroisochioliniumacetat.

Unter den gebräuchlichen Verfahren zur Racemtspaltung von optisch aktiven Aminen (Übersicht in: P. H. Boyle, Quart. Rev. *25* (1971) 323—341), wie z.B. der Diastereomerenbildung mit einem optisch aktiven Hilfsreagenz und deren Trennung, der Chromatographie an optisch aktiven Phasen oder des asymmetrischen Abbaus durch biologische oder chemische Methoden, ist die Spontankristallisation eines Enantiomeren oder einer seiner Abkömmlinge ein verhältnismäßig selten genutzes Verfahren [s. Collet et al.: Optical Resolution by Direct Crystallization of Enantiomer Mixtures; Chem.Rev. *80* (1980) 215—38].

Voraussetzung für eine direkte Kristallisation eines Enantiomeren aus seinem Racemat ist
a) die Kristallisation des Racemats als Konglomerat
b) eine geringere Löslichkeit der betreffenden Enantiomeren als die der Racematform
c) eine ohne Verzögerung erfolgende Kristallisation des jeweiligen Enantiomeren.

A priori ist es nicht möglich vorherzusagen, ob ein Racemat als Konglomerat kristallisiert und damit einer Trennung durch Spontankristallisation zugänglich ist. Unter den bekannten Beispielen (u.a. Collet et al., Bull. Soc. Chem. Fr. *1972* 127; id. ibid. *1977* 494) befinden sich hauptsächlich Aminocarbonsäuren, während die Anzahl reiner Amine sehr gering ist.

Es war daher umso überraschender, daß mit dem Acetat von (R,S)-11-(4-Methoxybenzyl)-octahydroisochinolin ein Salz gefunden wurde, das durch Spontakristallisation optisch getrennt werden kann.

Das zur Durchführung des erfindungsgemäßen Verfahrens benötigte 1-(4-methoxybenzyl)-octahydroisochinoliniumacetat kann in üblicher Weise leicht aus den Komponenten erhalten werden. Besonders vorteilhaft ist die Darstellung in situ durch Vereinigen äquimolarer Mengen 1-(4-Methoxybenzyl)-octahydroisochinolin und Eisessig in einem zur Ausführung der Spontankristallisation geeigneten Lösungsmittel.

Geeignete Lösungsmittel sind Wasser, Alkanole mit 1 bis 6 C-Atomen, Alkanone mit 3 bis 6 C-Atomen oder Gemische dieser Lösungsmittel. Bevorzugte Lösungsmittel sind Alkohole, insbesondere Isopropanol.

Übersättigte Lösungen an racemischem 1-(4-Methoxybenzyl)octahydroisochinoliniumacetat können in üblicher Weise nach bekannten Methoden erhalten werden, z.B. durch Konzentrierung, indem das Lösungsmittel verdampft wird, oder durch Versetzen mit einem weiteren Lösungsmittel. Am vorteilhaftesten ist es, eine heiß gesättigte Lösung des Acetats zu kühlen und diese so aufgrund der bei niedriger Temperatur geringeren Löslichkeit in den übersättigten Zustand zu überführen. Zur Herstellung der übersättigten Lösung ist es nicht erforderlich, ein 1:1-Gemisch der Enantiomeren zu verwenden, vielmehr ist es ein Vorteil, einen Antipoden im Überschuß vorliegen zu haben und diesen beim Abkühlen spontan auszukristallisieren.

Eine übersättigte Lösung von racemischem 1-(4-Methoxybenzyl)-octahydroisochinoliniumacetat wird dem erfindungsgemäßen Verfahren entsprechend mit einer geringen Menge an einem kristallinen Enantiomeren versetzt (Impfung). Dies kann derart geschehen, daß man diese Menge in der heißen übersättigten Lösung des Racemats auflöst und abkühlen läßt und so das zugesetzte Enantiomere spontan auskristallisieren läßt; oder die bereits erkaltete übersättigte Lösung wird mit einer geringen Menge an optisch einheitlichen Impfkristallen versetzt oder ein Teil der Impfkristalle wird in der Wärme gelöst und der Rest nach Erkalten auf Kristallisationstemperatur zugesetzt. Rühren erleichtert in der Regel die Kristallisation.

Der Temperaturbereich, in dem die Kristallisation durchgeführt wird, leigt vorzugsweise bei 0 bis 50°C, insbesondere bei annähernd 0°C.

Als der übersättigten Lösung zuzusetzende Menge an Impfkristallen sind 0,05 bis 5%, vorzugsweise 0,5 bis 5%, bezogen auf das Gewicht der Lösung, ausreichend. Die Impfkristalle sollten von hoher optischer Reinheit sein, um eine mögliche Mitkristallisation des unerwünschten Isomeren zu vermeiden. Nach Kristallisation und Isolierung durch z.B. Filtration oder Zentrifugieren eines Enantiomeren verbleibt in der Mutterlauge ein Überschuß des anderen Enantiomeren.

In einer weiteren bevorzugten Ausführungsform der Erfindung kann dieses andere Enantiomere derart erhalten werden, indem man die Mutterlauge wieder in einer in Bezug auf das Racemat übersättigte Lösung überführt. Dies kann entweder durch Konzentration der Mutterlauge geschehen, d.h. durch Verdampfen des Lösungsmittels, oder durch Zusatz einer bestimmten Menge an racemischem 1-(4-Methoxybenzyl)-octahydroisochinoliniumacetat. Vorzugsweise setzt man die Menge an Racemat zu, die derjenigen des abgetrennten Enantiomeren entspricht.

Die Ausführung der Spontankristallisation erfolgt in der gleichen Weise wie zuvor beschrieben mit der Ausnahme, daß nun die Impfung mit Kristallen des Antipoden des zuvor kristallierten Enantiomeren erfolgt. Diese Verfahrensweise läßt sich bevorzugt mehrfach weiderholen unter sukzessiver und abwechselnder Abtrennung der optisch aktiven Enantiomeren und Zugabe racemischer Verbindung.

Auf diese Weise kann racemischer (R,S)-1-(4-Methoxybenzyl)-octahydroisochinoliniumacetat einfach und vollständig in die Enantiomeren gespalten werden.

Erfindungsgemäß lassen sich aus den isolierten optisch aktiven 1-(4-Methoxybenzyl)-octahydroisochinoliniumacetaten leicht die freien Amine erhalten.

Die z.B. durch Filtration oder Zentrifugieren erhaltenen Kristalle an optisch aktivem (R)- oder (S)-Acetat werden durch Behandlung mit einer anorganischen Base in Freiheit gesetzt. Geeignete Basen sind alle hierfür überlicherweise verwendeten, z.B. Alkali- und Erdalkalihydroxide, -carbonate und -hydrogencarbonate sowie Ammoniak, starke organische Basen und basische Ionenaustauscher. Bevorzugte Basen sind Alkalihydroxide und Ammoniak, insbesondere Kalium- und Natriumhydroxid. Die Freisetzung des optisch aktiven 1-(4-Methoxybenzyl)-octahydroisochinolins erfolgt durch Zugabe der Base zu einer Lösung des optisch aktiven Acetats in einem geeigneten Lösungsmittel, vorzugsweise Wasser. Mittels eines organischen Loßungsmittels wie z.B. Diethylether, Toluol, Essigester, Dichlormethan oder auch deren Gemischen Läßt sich dann das freigesetzte Amin extrahieren. Gegebenenfalls nach Waschen des Extraktes wird das Lösungsmittel entfernt und so das gewünschte optisch aktive Amin erhalten.

In der vorliegenden Erfindung steht somit ein sehr vorteilhaftes Verfahren zur einfachen, vollständigen und in hoher Ausbeute, ohne Verwendung eines chiralen Hilfsreagenz durchführbaren optischen Spaltung von (R,S)-1-(4-Methoxybenzyl)-octahydroisochinolin zur Verfügung.

## Beispiel 1

Die Lösung von 300 g racemischem 1-(4-Methoxybenzyl)-octahydroisochinolin in 300 ml Isopropanol wird mit 67 ml Eisessig versetzt und nach Kühlen auf 0°C mit (S)-1-(4-Methoxybenzyl)-octahydroisochinoliniumacetat geimpft. Es entsteht ein dicker Kristallbrei, der abfiltriert und mit Diethylether ausgewaschen wird. Man erhält so 56,5 g (30,5% d. Th.) (S)-Acetat; F: 89,5°C; $[\alpha]_D^{20} = -118,7°C$ (c = 2% in Methanol).

Das Impfmaterial wird aus reinem (S)-1-(4-Methoxybenzyl)-octahydroisochinolin (3 g) in 8 ml tert.-Butylmethylether durch Versetzen mit Eisessig (0,67 ml) erhalten. Ausbeute: 3,49 g (94,3% d. Th.); F: 90°C, $[\alpha]_D^{20} = -119°$ (c = 2% in Methanol).

## Beispiel 2

300 g (R,S)-1-(4-Methoxybenzyl)-octahydroisochinolin werden in 300 ml Isopropanol geloßt und mit 67 ml Eisessig versetzt. Unter Rühren kühlt man auf 0°C ab und impft durch Zugabe von (R)-1-(4-Methoxybenzyl)-octahydroisochinoliniumacetat an. Bei dieser Temperatur wird 3 bis 5 Stunden langsam weiter gerührt. Man saugt das ausgefallene (R)-Acetat unter Nachwaschen mit tert.-Butylmethylether ab. Ausbeute: 88,8 g (48% d. Th.); F: 88—89°C; $[\alpha]_D^{20} = +116°$.

## Beispiel 3

In Isopropanol wird eine derartige Menge an racemischem (R,S)-1-(4-Methoxybenzyl)-octahydroisochinoliniumacetat gelöst, daß eine 52,6 Gew.%ige (R,S)-1-(4-Methoxybenzyl)-octahydroisochinoliniumacetatlösung entsteht. Die Lösung wird bei 20°C mit etwa 1% bezogen auf das Gewicht der Lösung an (S)-Acetat geimpft und 2 bis 3 Stunden bei 0°C gerührt. Das auskristallisierte (S)-

**EP 0 176 856 B1**

Acetat wird unter Nachwaschen mit tert.-Butylmethylether abgesaugt.

Durch Zugabe frischen (R,S)-Acetats zur Mutterlauge wird deren Konzentration wieder auf 52,6 Gew.% eingestellt. Zehnminütiges Erwärmen der Lösung auf 50°C löst etwa vorhandene Kristallkeime auf. Nun wird mit etwa 1%, bezogen auf das Gewicht der Lösung, (R)-Acetat angeimpft und 3 bis 4 Stunden bei 0°C gerührt. Das auskristallisierte (R)-Acetat wird unter Nachwaschen mit tert.-Butylmethylether abgesaugt.

Die Mutterlauge wird erneut durch Zugabe von (R,S)-1-(4-Methoxybenzyl)octahydroisochinolinium-acetat auf 52,6 Gew.% aufkonzentriert. Nachdem 10 Minuten auf 50°C erwärmt worden ist, läßt man abkühlen und impft an durch Zugabe von etwa 1%, bezogen auf das Gewicht der Lösung, an (S)-Acetat. Nach Absaugen und Waschen des auskristallisierten (S)-Acetats wird, wie oben beschrieben, die Mutterlauge durch Racematzugabe erneut aufkonzentriert und wieder mit (R)-Acetat angeimpft.

Die folgende Tabelle orientiert über die erzielten Resultate:

Enantiomeren-Isolierung

| getrennte Enantiomerenform | Zusätze (RS)-MBI-Acetat | Impf-material 0,90 g | Salz-Ausbeute g excl. Impf-Anteil | % | (S)-MBI-Acetat g excl. Impf-Anteil | Opt. Rein-heit % | Opt. Aus-beute % | (R)-MBI-Acetat g excl. Impf-Anteil | Opt. Rein-heit % | Opt. Aus-beute % | i-ProH Haupt-ML g | MTB Wasch-Konz. g | vereinigte Mutterlaugen Gehalte (S) Acetat g | (R) Acetat g |
|---|---|---|---|---|---|---|---|---|---|---|---|---|---|---|
| (S) | 74,0 | (S) | 9,56 | 12,9 | 9,56 | 97,6 | 25,2 | | | | 119,1 | 6,48 | 27,57 | 36,87 |
| (R) | 9,56 | (R) | 22,24 | 30,1 | | | | 22,24 | 97,1 | 51,8 | 98,3 | 9,2 | 32,05 | 19,70 |
| (S) | 22,24 | (S) | 21,33 | 28,8 | 21,33 | 94,4 | 46,6 | | | | 96,1 | 10,7 | 22,40 | 30,26 |
| (R) | 21,33 | (R) | 19,91 | 26,9 | | | | 19,91 | 97,8 | 47,6 | 99,2 | 9,65 | 9,65 | 21,20 |
| (S) | 19,91 | (S) | 19,52 | 26,4 | 19,52 | 98,2 | 44,8 | | | | 97,35 | 10,1 | 23,45 | 31,03 |
| (R) | 19,52 | (R) | 17,58 | 23,8 | | | | 17,58 | 97,9 | 42,2 | 103,7 | 9,15 | 33,06 | 23,36 |
| (S) | 17,58 | (S) | 12,28 | 16,6 | 12,28 | 98,2 | 28,8 | | | | 111,8 | 7,6 | 29,64 | 31,89 |
| (R) | 12,28 | (R) | 11,9 | 16,1 | | | | 11,90 | 98,3 | 30,5 | 114,2 | 6,7 | 35,55 | 26,55 |
| (S) | 11,9 | (S) | 10,86 | 14,7 | 10,86 | 97,6 | 25,5 | | | | 115,0 | 6,95 | 30,73 | 32,40 |
| (R) | 10,86 | (R) | 11,46 | 15,5 | | | | 11,46 | 97,6 | 29,5 | 114,0 | 7,3 | 36,01 | 26,52 |
| (S) | 11,46 | (S) | 13,84 | 18,7 | 13,84 | 98,0 | 32,5 | | | | 108,8 | 7,6 | 28,00 | 32,15 |
| (R) | 13,84 | (R) | 10,7 | 14,5 | | | | 10,7 | 97,6 | 26,7 | 117,3 | 6,3 | 34,82 | 28,49 |
| (S) | 15,48 | (S) | 15,48 | 20,9 | | 98,4 | 37,4 | | | | | | 25,36 | 33,16 |
| (R) | 22,40 | (R) | 22,40 | 30,3 | | | | | 92,8 | 50,7 | | | 32,30 | 19,31 |

[MBI.Ac = 1-(4-Methoxybenzyl)-octahydroisochinoliniumacetat]

EP 0 176 856 B1

## EP 0 176 856 B1

Konzentration konstant 74,0 g (RS)-MBI-Acetat (+ 0,9 g Impfmaterial) in 141,5 g i-PrOH-Lsg. Ergänzen der abgetrennten Enantiomeren durch frisches (RS)MBI-Acetat. 1,2% Impfmaterial. Zugabe bei 20°C. Vorherige Entkeimung 10 Min/50°C. Kristallisationszeiten: (S)-Stufe 2 1/2 h/O°C; (R)-Stufe 3 1/2 h/O°C.

Beispiel 4

Die Lösung von 31,7 g (S)-1-(4-Methoxybenzyl)-octahydroisochinoliniumacetat in 250 ml Wasser wird durch Zugabe von konzentrierter Natronlauge auf pH 12 gebracht. Mit zweimal je 150 ml Diethylether wird die freie Base extrahiert. Nach Waschen der vereinigten Extrakte mit Wasser, Trocknen über Natriumsulfat und Entfernen des Lösungsmittels im Vakuum hinterbleiben 25,0 g (97,3% d.Th.) (S)-1-(4-Methoxybenzyl)-octahydroisochinolin.

### Patentansprüche

1. (R,S)-1-(4-Methoxybenzyl)-1,2,3,4,5,6,7,8-octahydroisochinoliniumacetat

2. (R)-1-(4-Methoxybenzyl)-1,2,3,4,5,6,7,8-octahydroisochinoliniumacetat

3. (S)-1-(4-Methoxybenzyl)-1,2,3,4,5,6,7,8-octahydroisochinoliniumacetat.

4. Verfahren zur Herstellung von optisch aktivem 1-(4-Methoxybenzyl)-1,2,3,4,5,6,7,8-octahydroisochinolin bzw. dessen Salzen durch fraktionierte Kristallisation der enantionmeren Salze aus einer Lösung des Racemats dieser Salze und gegebenenfalls Freisetzung der Base, dadurch gekennzeichnet, daß man die übersättigte Lösung von racemischem 1-(4-Methoxybenzyl)-octahydroisochinoliniumacetat mit seinen enantionmeren Formen impft und das der Konfiguration der Impfkristalle entsprechende optisch aktive Acetat kristallisiert.

5. Verfahren nach Anspruch 4, dadurch gekennzeichnet, daß man die Impfkristalle in einer Menge von 0,05 bis 5%, bezogen auf das Gewicht der übersättigten Lösung, zusetzt.

6. Verfahren nach einem der Ansprüche 4 und 5, dadurch gekennzeichnet, daß man in der nach der Abtrennung eines betreffenden Enantiomeren erhaltenen Mutterlauge zusätzliches racemisches 1-(4-Methoxybenzyl)-octahydroisochinoliniumacetat bei erhöhter Temperatur löst unter Erhalt einer weiteren übersättigten Lösung und aus dieser das andere Enantiomere kristallisiert.

7. Verfahren nach Anspruch 6, dadurch gekennzeichnet, daß man die Verfahrensweise mehrfach wiederholt unter sukzessiver und abwechselnder Abtrennung der optisch aktiven enantiomeren 1-(4-Methoxybenzyl)-octahydroisochinoliniumacetate.

### Revendications

1. Acétate de (R,S)-1-(4-méthoxybenzyl)-1,2,3,4,5,6,7,8-octahydroisoquinolinium.

2. Acétate de (R)-1-(4-méthoxybenzyl)-1,2,3,4,5,6,7,8-octahydroisoquinolinium.

3. Acétate de (S)-1-(4-méthoxybenzyl)-1,2,3,4,5,6,7,8-octahydroisoquinolinium.

4. Procédé de préparation de 1-(4-méthoxybenzyl)-1,2,3,4,5,6,7,8-octahydroisoquinoléine optiquement active ou de ses sels par cristallisation fractionnée des sels énantiomères à partir d'une solution du racémate de ces sels et le cas échéant libération de la base, caractérisé en ce qu'on ensemence la solution sursaturée d'acétate de 1-(4-méthoxybenzyl)-octahydroisoquinolinium racémique avec ses formes énantiomères et en ce qu'on cristallise l'acétate optiquement actif correspondant à la configuration des cristaux d'ensemencement.

5. Procédé selon la revendication 4, caractérisé en ce qu'on ajoute les cristaux d'ensemencement en une quantité de 0,05 à 5% par rapport au poids de la solution sursaturée.

6. Procédé selon l'une des revendications 4 et 5, caractérisé en ce qu'on dissout à température élevée l'acétate de 1-(4-méthoxybenzyl)-octahydrosioquinolinium racémique supplémentaire dans la liqueurmère obtenue après séparation d'un énantiomère considéré en obtenant une autre solution sursaturée, à partir de quoi on cristallise l'autre énantiomère.

7. Procédé selon la revendication 6, caractérisé en ce qu'on repete ce procédé plusieur foix pendant la separacion successive et alternée d'acétate de 1-(4-méthoxybenzyl)-octahydroisoquinolinium optiquement actif.

### Claims

1. (R,S)-1-(4-methoxybenzyl)-1,2,3,4,5,6,7,8-octahydroisoquinolinium acetate.

2. (R)-1-(4-methoxybenzyl)-1,2,3,4,5,6,7,8-octahydroisoquinolinium acetate.

3. (S)-1-(4-methoxybenzyl)-1,2,3,4,5,6,7,8-octahydroisoquinolinium acetate.

4. Process for preparing optically active 1-(4-methoxybenzyl)-1,2,3,4,5,6,7,8-octahydroisoquinoline and its salts by fractional crystallisation of the enantiomeric salts from a solution of the racemate of these salts and, if desired, freeing the base, characterised in that in that the supersaturated solution of racemic 1-(4-methoxybenzyl)-octahydroisoquinolinium acetate is seeded with its enantiomeric forms and the optically active acetate which corresponds to the configuration of the seed crystals is crystallised.

5. Process according to Claim 4, characterised in that the seed crystals are added in an amount of 0.05 to 5%, relative to the weight of the supersaturated solution.

6. Process according to either of Claims 4 and 5, characterised in that additional racemic 1-(4-methoxybenzyl)-octahydroisoquinolinium acetate is dissolved at elevated temperature in the mother liquor obtained after seperating off an enantiomer of the type in question to obtain a further supersaturated solution and from this solution the other enantiomer is crystallised.

7. Process according to Claim 6, characterised in that the procedure is repeated a plurality of times with successive and alternate separating off of the optically active enantiomeric 1-(4-methoxybenzyl)-octahydroisoquinolinium acetates.